# EUROPEAN PATENT SPECIFICATION

(11) **EP 1 572 257 B1**
(45) Date of publication and mention of the grant of the patent: **13.09.2006**
(21) Application number: 03813131.4
(22) Date of filing: 16.12.2003
(51) Int. Cl.: A61L 27/10

(54) **BONE SUBSTITUTE MATERIAL**
KNOCHENERSATZMATERIAL
MATIERE OSSEUSE DE SUBSTITUTION

(30) Priority: 16.12.2002 DE 10258773
(43) Date of publication of application: 14.09.2005
(73) Proprietor: SDGI Holdings, Inc., Wilmington, DE 19801 (US)
(72) Inventor: RICHART, Olivier, F-13340 Rognac (FR); MALZER, Wolfgang, 94469 Deggendorf (DE)
(74) Representative: Viering, Jentschura & Partner
(86) International application number: PCT/EP2003/014338
(87) International publication number: WO 2004/054633

(56) References cited:
- EP-A- 0 061 108
- EP-A- 0 987 032
- US-A- 5 147 904
- US-A- 5 766 618
- US-B1- 6 316 091

## Description

The invention relates to the manufacturing of bone substitute material as well as to methods for producing such bone substitute material and to macroporous synthetic ceramic material for the production of bone substitute material.

In bone surgery, particularly vertebral column surgery, implants are used for the replacement of bone bodies, particularly like dorsal vertebrae, or bone bodies are also used to replace intervertebral discs to safely strut adjacent dorsal vertebrae. It is an important aspect that such implants quickly conjoin with the surrounding bone material, i.e. that the surrounding bone material grows into the implant in order for the latter to stabilise in its position as soon as possible in the form of a bone-implant-compound.

Therefore, e.g. plastic implants are known which comprise a hollow space into which genuine bone material is filled in order for the surrounding bone material to grow through the hollow space through the implant to thus achieve a stabilisation of the implant (cf. e.g. implant CORNERSTONE-SR™ of Medtronic Sofamor Danek). Also known are genuine bone material implants (cf. e.g. DE 35 05 567 A1).

Furthermore, e.g. from US 6,316,091 B1 a macroporous synthetic ceramic bone implant is known where the ceramic body is intentionally provided with macropores for the surrounding bone material to better grow into the implant to thus improve the stabilisation of the implant in its position.

EP-A-061 108 discloses bone implant material consisting of a tricalciumphosphate ceramic with a porosity grade of more than 50%, which, in form of a granulate, is submitted to a heat treatment in order to increase its uptake of an active substance such as an antiseptic.

US-A-5 766 618 discloses the manufacture of macroporous polymer matrices for use as bone graft of implant material, said composites being formed from a calcium phosphate ceramic and hydroxyapatite.

EP-A-987 032 discloses the manufacture of an osteoinductive biomaterial based on a macroporous ceramic material, wherein the ceramic material preferably is a calcium phosphate and the macropores are interconnected.

It is an object of the invention to provide for a production of bone substitute material, with which bone substitute material sufficiently stable bone bridges can be achieved in a fast and safe manner and which bone substitute material being biocompatible and applicable in many ways. Furthermore, the invention provides a method for the production of such bone substitute material and an implant provided with such bone substitute material.

According to the invention, this is achieved on the one hand by the use of a macroporous synthetic ceramic, which is produced by introducing organic compound particles into a container, subjecting the particles to a thermoforming process, thereby melting together the particles to provide a monobloc structure of interconnected particles with hollow spaces therebetween, impregnating the monobloc structure with a calcium phosphate suspension, thereby filling up the spaces in the monobloc strucutre, removing the liquid from the suspension on the monobloc structure, removing the monobloc structure to form a ceramic material in form of a compound of interconnected hollow bodies, the hollow spaces of which being connected by macropores, and sintering the ceramic material so as to obtain the macroporous synthetic ceramic, for producing granular bone substitute material.

The invention is based upon the perception that macroporous synthetic ceramic produced in this way can easily be processed into a granulate very suitable for the use as bone substitute material. This suitability results from, besides, a granulate of this material can be produced with particles having such a dimension, for example with respectively a mean particle diameter of 0,5-5mm, to fast and with sufficient spaces fill a hollow space, like for example in an implant like the aforementioned Cornerstone-ST™, or in a bone, in order for the surrounding bone material grow into the granulate fast and guaranteeing a high stability. In this respect, the granulate particles themselves are very suitable for the bone material to grow through them owing to them including interconnected macropores, and in addition they are very biocompatible. Additionally, the granulate particles have sufficient mechanical strength with regard to pressure and friction forces for not being pulverised by the mechanical impacts in the implanted state. Thus, the granulate which can be obtained according to the invention and which is made of the macroporous synthetic ceramic as described above can be applied in many ways in combination with different implants or alone in order to obtain bone bridges.

A macroporous synthetic ceramic like the aforementioned is for instance described in US 6,316,091 B1 mentioned above.

Preferably, the thermoforming process happens at a temperature above the glass transition temperature, the organic component can be removed from the surrounding ceramic component for instance simply by melting it out via heating the compound of monobloc structure and ceramic as it has a melting temperature lower than the ceramic component.

Preferably, the thermoforming process is achieved by homogeneous heating of the organic component particles at a temperature above the glass transition temperature. In particular, thermoplastic polymers come into consideration as preferred organic component materials, like for instance polymethyl metacrylate (PMMA), polymethacrylate (PMA), polystyrene, polyethylene and compounds thereof.

In this connection, the organic component preferably at least in part has an amorphous structure.

The single organic component particles have a preferred size of 0.2 to 0.7mm, especially preferred 0.4 to 0.6mm mean diameter, wherein the respective monobloc structure is preferably compounded of particles of equal size. In the manufacturing process the particles for instance are filled into a heated container where they are subjected to the thermoforming process and are thermally fused together to provide the monobloc structure and then cooled to environmental temperature. Then the thus resulting monobloc structure for instance arranged on a plaster plate, can be impregnated with the suspension which completely covers the monobloc structure and also fills the spaces therein. The plaster plate itself is preferably arranged in an elastic mould, such as a silicone mould. The liquid contained in the suspension is absorbed by the plaster plate so that an overall compact compound of the monobloc structure and the encasing hardened ceramic is formed. The elasticity of the mould allows easy removing of this compact compound which is then relieved of the organic constituent of the monobloc structure by melting out said organic constituent so that in the end only the negative of the monobloc structure remains, which, by sintering, becomes the macroporous synthetic ceramic. This macroporous synthetic ceramic comprises macropores at the former interconnections between the particles of the monobloc structure, preferably within a range of 100µm to 800µm, especially preferably from 100µm to 150 µm diameter, and besides, constitutes a compound of adhering, particularly round, as almost spherical, hollow bodies (according to the outside shape of the particles of the monobloc structure).

Preferably, the suspension comprises hydroxyapatite or tricalciumphosphate or a compound thereof.

Preferably, the container is made of metal, plaster or a polymere material or a compound thereof.

In particular, the organic component is being removed at a temperature of 1000-1300 °C.

According to the invention, the granulate manufactured from the macroporous synthetic ceramic preferably comprises particles having a mean diameter of at least 0,5mm and preferably a maximum of 6mm. Within this range, the granulate, having the form of a bulk material, is particularly receptive for surrounding bone material, which is to grow into it and through it. In addition, such big and nevertheless sufficiently strong particles can be obtained by the aforementioned synthetic ceramic without any problem.

Although the granular bone substitute material can be made of the respective macroporous synthetic ceramic for instance by cutting which then results in substantially defined outline surfaces, it is preferably used for producing granular bone substitute material with broken particles having at least partly, but preferred, substantially all-side irregular outside surfaces.

Such a granular bone material, having only partly broken outside surfaces, can for instance be made by the respective macroporous synthetic ceramic being formed so that the suspension assumes such an outside shape, that already after the forming process, the hardened suspension constitutes a multitude of particles interconnected by predetermined breaking points which then can be separated simply by breaking at the said breaking points. For this, for instance, already the monobloc structure particles are melted together in such a form, whereupon subsequently the suspension is being introduced.

According to the invention, the method for producing bone substitute material, in particular the aforementioned bone substitute material, comprises: introducing particles of an organic compound into a container, subjecting the particles to a thermoforming process thereby melting the particles together to provide a monobloc structure of interconnected particles with hollow spaces therebetween, impregnating the monobloc structure with a calcium phosphate suspension, thereby filling up the spaces in the monobloc structure, removing the liquid from the suspension on the monobloc structure, removing the monobloc structure to form a ceramic material in form of a compound of interconnected hollow bodies, the hollow spaces of which being connected by macropores, sintering the ceramic material so as to provide a macroporous synthetic ceramic, and fracturing the synthetic ceramic gained with this way to particles to a granulated material serving as bone substiture material. Preferably, the synthetic ceramic is produced in block or sheet form, wherein said block or sheet formed synthetic ceramic is transformed into a granulated material, serving as bone substitute material, by fracturing it on and passing it through a first sieve with a first mesh size, and then passing these fractured particles through a second sieve with a second smaller mesh size, the particles selected from the second sieve being used as granulate material. The synthetic ceramic is manufactured by, in detail, preferably proceeding as described above.

The breaking of the synthetic ceramic block or the synthetic ceramic sheet, for instance, can happen immediately on the first sieve, for instance by working, rubbing and pounding, the ceramic lying on the first sieve, with a brush part comprising protruding teeth. For this, for instance, above the first sieve, also additionally, a third sieve can be arranged with a mesh size larger than the mesh size of the first sieve, and on which the ceramic is broken.

By this method, granulate particles are being prepared with substantially all-side irregular surfaces and therefore, particularly, being altogether irregular, i.e. ceramic particles being irregular in terms of shape. Then these particles, filled into an implant, provide for a correspondingly irregular and nevertheless sufficiently compact but not powdery bulk material.

By the process mentioned, particles are obtained which have approximately the same mean perimeter diameter but it is also conceivable to produce granulates with particles of different sizes for use as a bone substitute material.

Alternatively, to achieve an as high as possible rate of yield in producing same size particles, according to the invention, the synthetic ceramic is produced in form of a plate, the surface of which on at least one of its plate sides being provided with a regular or irregular grooved pattern, and wherein the plate is fractured along the grooves into particles thus to obtain a granulate material to serve as bone substitute material. Further alternatively, the synthetic ceramic mentioned is produced in form of a block having a three-dimensional regular or irregular grid of perforation lines, along which the block is fractured into particles, thus obtaining a granulated material to serve as bone substitute material. Further alternatively, the synthetic ceramic mentioned is being produced in the form of a grid body with regular or irregular grid elements, and wherein the grid body is fractured into particles, thus providing a granulate material to serve as bone substitute material. In the latter case, the grid body is preferably produced as two-dimensional grid, like particularly a grid sheet.

When preparing the macroporous synthetic ceramic mentioned, it can simply be formed in the structures stated. Thus, for instance, for the preparation of the grid sheet, a corresponding negative can be included in a side wall of a container, in which the monobloc structure is formed and subsequently filled with the suspension. As well, a shaping mat can be provided with a negative corresponding to the grid, which is filled with the organic component particles, thereby filling the hollow spaces which later form the grid elements, wherein then this mat is lodged in a heating oven for thermoforming the particles and subsequently the monobloc structure in the shaping mat is contacted with the suspension. Preferably, such a mat is made of elastic synthetic material in order to facilitate its separating from the ceramic grid element with the integrated monobloc structure.

The synthetic ceramic sheet can be produced, for instance, at first in a corresponding form with no outside grooves, wherein the grooves then subsequently are prepared by carving, with a pointed object, into the flat sheet. But also, the grooves can be formed by a corresponding shaping of the container wall, in which the suspension is filled, or a separate plastic or metal grid can be inserted into the form into which the suspension is filled as well and cast together with the suspension and the monobloc structure. Such a grid can for instance be a metal or plastic thread woven material having a large mesh size. When the grid is being removed from the monobloc structure and ceramic suspension compound, it remains a synthetic ceramic sheet part provided with a corresponding surface pattern from which then by heating, the monobloc structure is melted out.

The same applies to the block shaped synthetic ceramic in which such a three-dimensional grid part can, at the same time, also be included; then this can, particularly, for instance as well be made of the same material as the monobloc structure and, together with it, melted out from the complete block. Alternatively, the grid part can also remain in the block shaped synthetic ceramic and for instance, be made of a material that can be separated easily from the synthetic ceramic.

Sheets or blocks provided with such tracing lines can then easily be broken to particles along these tracing lines serving as predetermined breaking points, wherein the irregular but preferably regular, as needed, particle size is pre-adjustable, preferably cube or parallelepiped shaped.

For the preparation of preferred cube sizes with an outside edge length of 0.5-6mm, preferably synthetic ceramic sheets with a corresponding thickness of 0.5-6mm are being used.

According to the invention, as intermediate product of the aforementioned methods, also macroporous synthetic ceramic materials are provided, wherein the synthetic ceramic material is produced in the aforementioned way according to the invention, and wherein according to a first alternative the synthetic ceramic material is produced in the form of a sheet the surface of which is provided with a regular or irregular groove pattern on at least one side of the sheet, according to a second alternative the synthetic ceramic material is manufactured in the form of a block which is inside provided with a three-dimensional regular or irregular grid of perforation lines, and according to a third alternative, the synthetic ceramic material is produced in the form of a grid body having regular or irregular grid elements, wherein the grid body preferably is a two-dimensional plate-shaped grid.

In the latter case, the grid elements in cross-section preferably have a medium diameter of 0.5-3mm; the mesh size in perimeter diameter is preferably 2-7mm.

The aforementioned materials can be sheet or block shaped, wherein the surgeon, as required at each case, for instance as well manually, breaks off granulated bone substitute material from the materials in order to fill, as required at each time, hollow spaces in the implants or bones through and/or into which surrounding bone matter shall grow.

According to the invention, further an implant is provided, in particular a spinal column implant, like for instance an intervertebral implant, having a hollow space within granular bone substitute material as described above according to the invention is accommodated.

According to the invention, henceforth the surgeon can approach in various ways. In this respect, according to the invention, the surgeon can insert the granulate material during the operation, before or after implanting the implant and/or can also fill up other spaces, like for instance cavities generated by drilling into the bone, in order to generate a loose bone bridge enabling the bone material to grow faster in or through to thus accelerate the stabilisation of the bone structure.

According to the invention, here the surgeon can chose the granulated bone substitute material appropriate in each special case immediately during the operation, from various granulated implant material at his disposal. I.e., the surgeon can decide immediately when operating whether to use for instance regular or irregular granulate material or a mixture of regular or irregular material.

But the surgeon can as well prepare an implant prior to the operation, i.e. for instance fill up said implant with the granulate material according to the invention. But as well, alternatively implants prepared in this way can be final products, i.e. be thus prepared by the supplier.

When using grid shape and/or perforated block- and/or sheet shaped bone substitute material the surgeon can, according to the invention, for instance immediately during the operation, break from the grid, block and/or sheet portion, bone substitute material in the required size of parts, however, in the case of sheet and block material in the minimum size substantially defined by the perforation lines, to fill the implant therewith and/or to build another loose bone bridge.

On the basis of preferred embodiments with reference to the drawings the invention is described as follows. In the drawings it is shown in:
Figure 1 a perspective view of a monobloc structure for the production of a macroporous synthetic ceramic for use in the production of granulated bone substitute material according to the invention,
Figure 2 a perspective view of a macroporous synthetic ceramic for use in the production of granulated bone substitute material according to the invention,
Figure 3 a perspective view of a mould for the production of synthetic ceramic sheet material according to the invention,
Figure 4 a macroporous synthetic ceramic material in sheet form to be used for the production of granulated bone substitute material according to the invention,
Figure 5 a macroporous synthetic ceramic according to the invention,
Figure 6 a woven wire grid to be used in the production of macroporous synthetic ceramic material according to the invention,
Figure 7 a structured mat to be used in the production of a macroporous synthetic ceramic material according to the invention,
Figure 8 a perspective view of macroporous synthetic ceramic material according to the invention,
Figure 9 an irregular granulate of macroporous synthetic ceramic according to the invention,
Figure 10 regular granulate of macroporous synthetic ceramic according to the invention, and
Figure 11 irregular granulate of macroporous synthetic ceramic according to the invention.

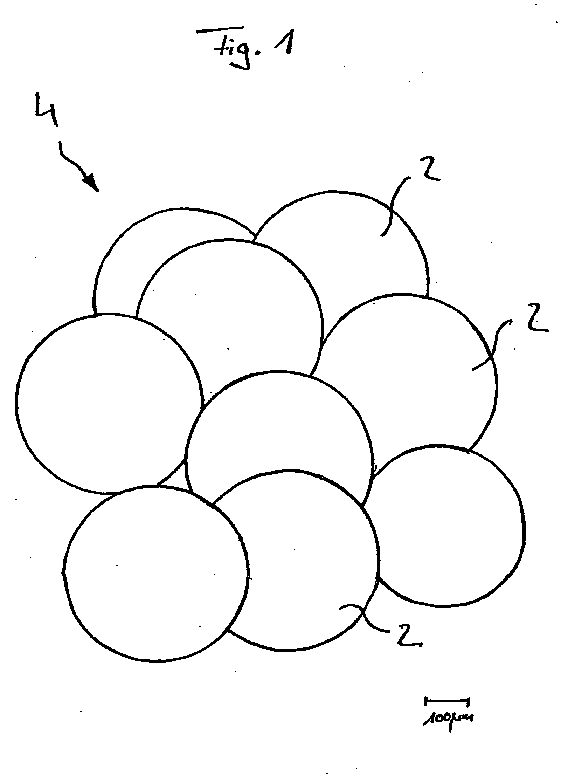

According to Figure 1, a plurality of round-shaped, here substantially spherical particles 2 are thermally melted together, thereby forming a cohesive monobloc structure 4, wherein the connections between the respective particles 2 in cross-section substantially have the same size. The single particles 2 are of thermoplastic full material, like for instance PMMA or PMA.

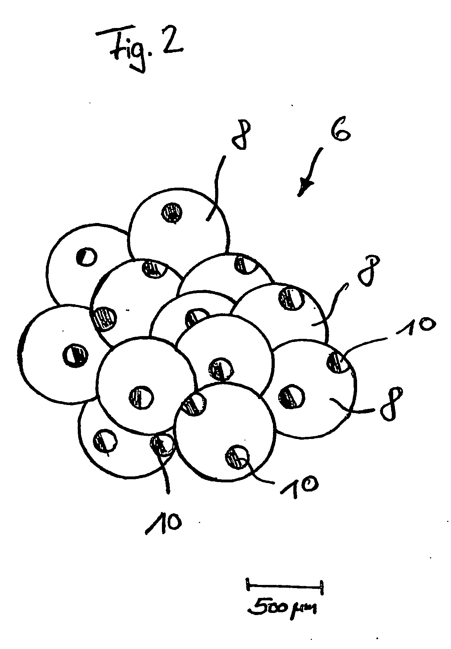

A synthetic ceramic produced on the basis of this monobloc structure 4 according to the invention is shown in Figure 2 in perspective view. The synthetic ceramic 6 according to Figure 2 provides the negative of the monobloc structure 4 shown in Figure 1, i.e. it comprises hollow round-shaped, here substantially spherical bodies 8 and holes 10 in formed therein. The hollow bodies 8 are interconnected with each other, thereby forming an overall structure (= synthetic ceramic 6).

The synthetic ceramic 6 is obtained by completely covering the monobloc structure 4 shown in Figure 1 by a suspension on the basis of calcium phosphate, like for instance hydroxyapatite (HA) or tricalciumphosphate (TCP), wherein as well the spaces in the monobloc structure 4 are filled, and after the removal of the liquid from the suspension the monobloc structure 4 is being removed, particularly by melting out the thermoplastic material from the ceramic material. Then the latter is sintered to finally become the synthetic ceramic shown in Figure 2.

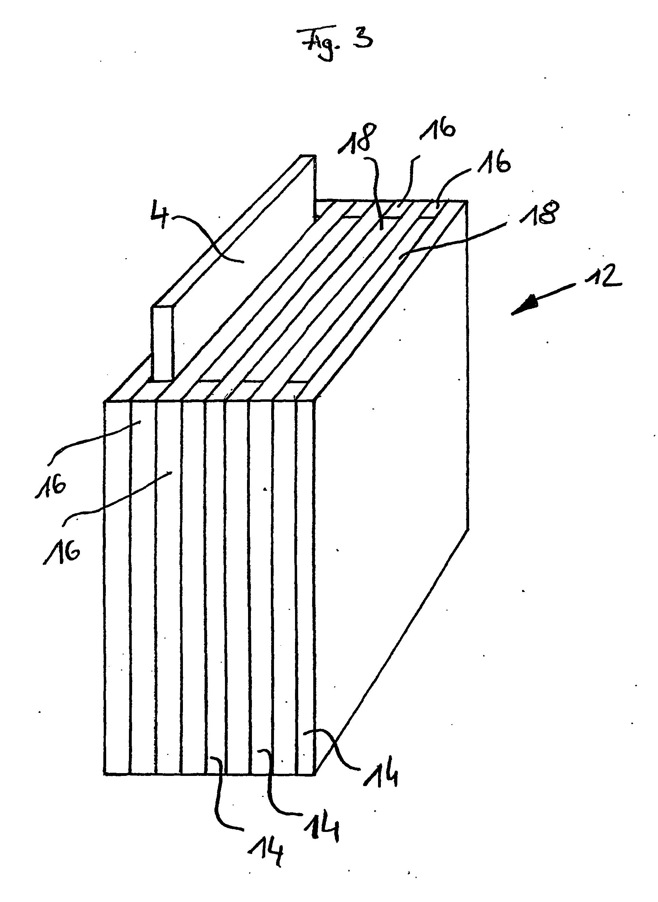

In Figure 3 a manufacturing device in the form of an electrically heated form 12 is shown in perspective view comprising a plurality of parallel plates 14 of a material with a good heat conduction quality, as in particular metal material, arranged in a respective distance with respect to each other via strip shaped spacers 16 which in each case are arranged edge-sided between the plates 14. The spacers 16 are also of a material of good heat conductivity, like particularly metal. The package of plates 14 and spacers 16 is firmly, but preferably, detachably, interconnected.

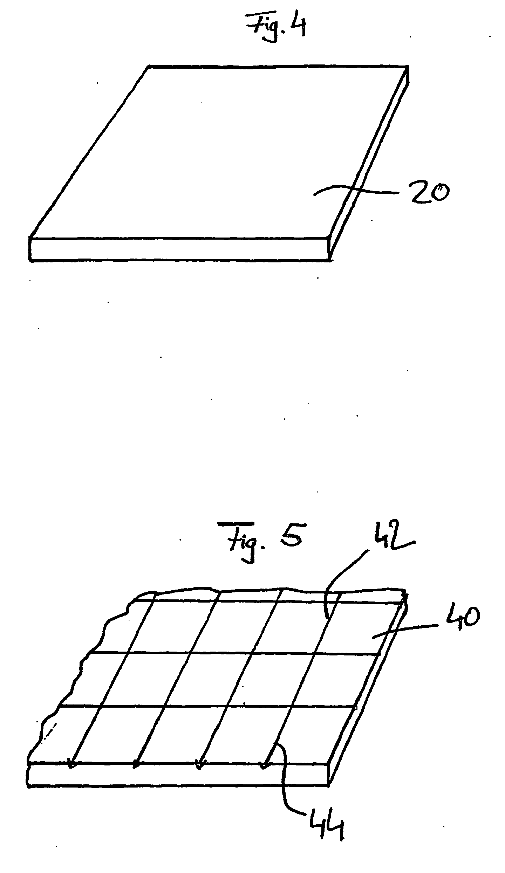

Therefore, large area, narrow hollow spaces 18 are situated between adjacent plates 14, into which firstly particles 2 of thermoplastic material (cf. Fig. 1) are being filled and subsequently, by a homogenous heating of the whole sheet compound, subjected to a thermoforming process thereby fused together to provide a monobloc structure. Subsequently, the sheet-shaped monobloc structures 4 generated in this way can be removed from the hollow spaces 18 and impregnated with the suspension, for instance, arranged on a plasterboard which itself is arranged in a preferably elastic form. When the liquid evaporates from the suspension filled sheets, bodies in the form of sheets evolve which correspond, in terms of form, to the corresponding sheet-shaped monobloc structure 4, the way it is shown for instance in Figure 3, partly pulled out of the hollow space 18. Then such a sheet-shaped body is heated, for instance also in a sheet packet so that the thermoplastic material of the monobloc structure 4 melts and drips from the hollow space 18 the way that finally a macroporous synthetic ceramic material 20 remains in the plate form, like it is shown for instance in Figure 4.

According to the invention, such a sheet-shaped macroporous synthetic ceramic material 20 can for instance be processed into granulate using a granulate production arrangement 22 as shown in Figure 12. Here for instance the sheet-shaped synthetic ceramic material 20 is being treated by rubbing and pounding on a first sieve 28 with a first mesh size, for instance with a nailbrush 24 with teeth 26 shown there, into the direction of sheet 20, wherein the particles passing through the first sieve 28 are directed onto a second sieve 30 with a mesh size smaller than that of the first sieve 28.

Under the second sieve a pan 32 is arranged in which the particles passing through the sieve 30 are being collected. The latter particles are of different sizes and can be used as a granulate which consists of different size particles. The particles remaining in the second sieve 30 are discharged as yield and serve as granulate consisting of particles with substantially the same mean outside diameter. The mesh size (one edge length or the perimeter diameter of the respective mesh) of the second sieve 30 amounts to preferably 0.5-6mm, wherein then the mesh size of the first sieve 28 in each case is preferably ca. 1mm bigger.

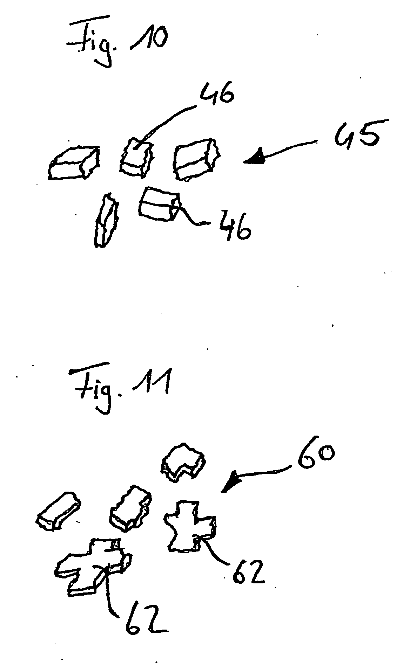

In Figure 9, a granulate 34 according to the invention is shown in schematic view, with irregular granulate particles 36 prepared on the basis of the arrangement 22 shown in Figure 12.

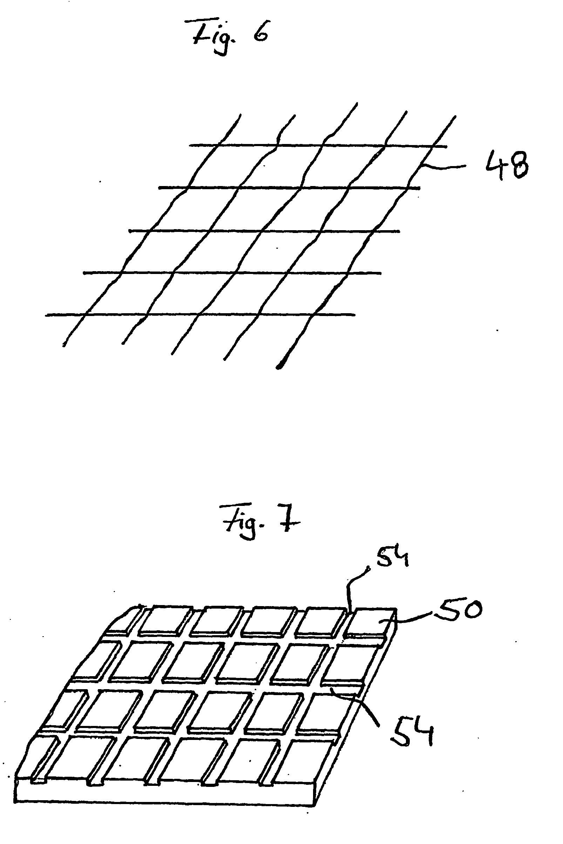

Figure 5 shows a macroporous synthetic ceramic material in sheet form 40 for the preparation of granulated bone substitute material.

Contrary to the flat sheet-shaped synthetic ceramic material 20, as exhibited in Figure 4, this synthetic ceramic material has a regular groove pattern 42 on one sheet side, wherein squares of equal sizes are being formed by the crossing grooves 44. Along the grooves 44 serving as predetermined breaking lines, the synthetic ceramic material 40 can simply be broken to parallelepiped shaped granulate particles. Here, it is particularly simple to generate cubes in that the sheet thickness corresponds to the edge length of the respective square.

In Figure 10 a granulate 45 according to the invention is shown, having parallelepiped shaped granulate particles 46 of equal sizes, the way they are for instance produced, as shown in Figure 5, on the basis of the synthetic ceramic material 40 (then with a respective rectangular pattern).

The grooves 44 can for instance be inserted by subsequent carving with a pointy object into a sheet form synthetic ceramic as shown in Figure 4; but also, the grooves 44 can be generated by the monobloc structure 4 already being provided with such grooves, for instance by corresponding strip-shaped rips being provided in the side plates 14 of the form 12 shown in Figure 3. In this respect, the grooves 44 can also be completely filled with suspension, wherein subsequently unwanted suspension material is being removed from the grooves in the ceramic. Also, on the opposite sheet side a groove pattern corresponding to groove pattern 42 can be formed congruently in order to further facilitate the accurate breaking of the particles.

As an alternative to a pattern being provided in the plates 14 of the preparation form 12 directly, a wire grid 48 (here woven) as shown for instance in Figure 6 can as well be inserted into the hollow space 18 of the form 12 shown in Figure 3 and thus form a groove pattern in the monobloc structure 4 which then is applied to the suspension.

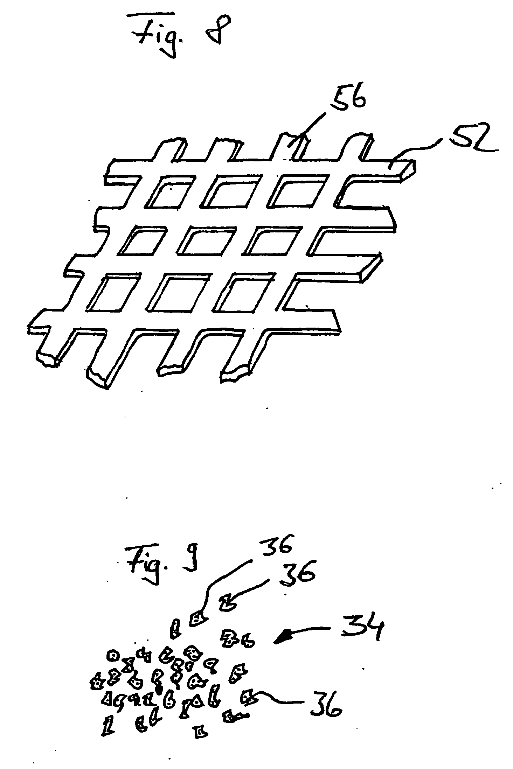

In Figure 7 a plastic mat 50 of flexible plastic material is shown which, according to the invention, is used for the manufacture of a grid shaped, macroporous synthetic ceramic material 52 for instance as exhibited in Figure 8. For this, the plastic mat 50 shows the negative of the grid 52 exhibited in Figure 8 in the form of respective grid shaped channels 54, into which organic component particles 2 (cf. Fig. 1) for the formation of a respective grid shaped monobloc structure 4 are being filled and heated in order for thermoforming. In this case, the mat 50 with particles 2 filled into the channels 54 for instance can be inserted into a form 12 as shown in Figure 3, i.e. into its hollow spaces 18. A ceramic compound produced in the aforementioned way (= suspension after removal of liquid) and monobloc structure 4 will then be removed from the mat 50 by for instance simply pulling off the latter. Subsequently, the monobloc structure 4 is being melted out from the ceramic, whereby finally the grid shaped synthetic ceramic material 52 according to the invention is generated as shown in Figure 8.

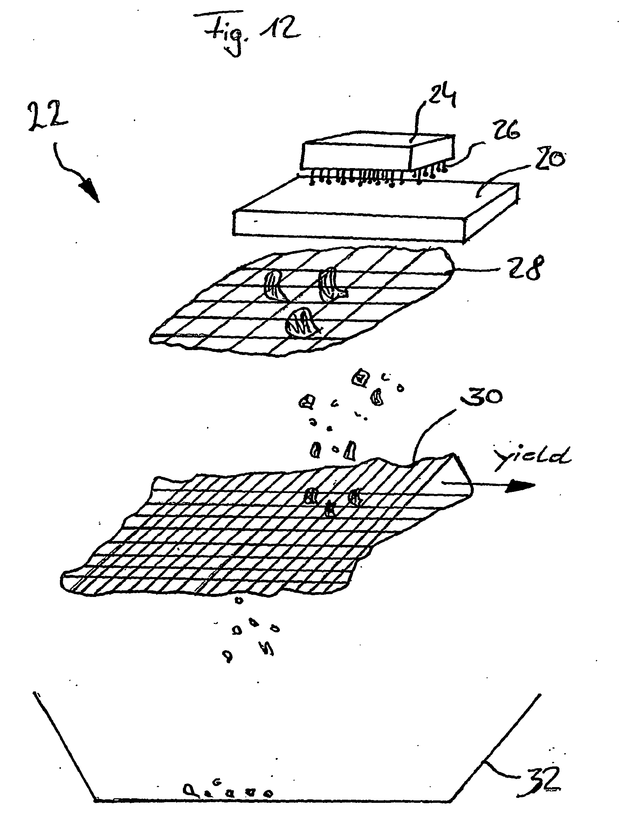

The latter is rather fragile owing to its rather large mesh in comparison to the grid elements 56, and can therefore easily be broken to a granulate form bone substitute material 60 comprising irregular, i.e. irregular in terms of form, granulate particles 62 having different sizes as shown for instance in Figure 11.

## Claims

1. Use of a macroporous synthetic ceramic which is produced by introducing particles of an organic compound into a container, subjecting the particles to a thermoforming process thereby melting the particles together to provide a monobloc structure of interconnected particles with hollow spaces therebetween, impregnating the monobloc structure with a calcium phosphate suspension, thereby filling up the spaces in the monobloc structure, removing the liquid from the suspension on the monobloc structure, removing the monobloc structure to form a ceramic material in form of a compound of interconnected hollow bodies, the hollow spaces of which being connected by macropores, and sintering the ceramic material so as to obtain the macroporous synthetic ceramic, for producing granular bone substitute material:

2. A method for producing bone substitute material, comprising: introducing particles of an organic compound into a container, subjecting the particles to a thermoforming process thereby melting the particles together to provide a monobloc structure of interconnected particles with hollow spaces therebetween, impregnating the monobloc structure with a calcium phosphate suspension, thereby filling up the spaces in the monobloc structure, removing the liquid from the suspension on the monobloc structure, removing the monobloc structure to form a ceramic material in form of a compound of interconnected hollow bodies, the hollow spaces of which being connected by macropores, sintering the ceramic material so as to provide a macroporous synthetic ceramic, and fracturing the synthetic ceramic gained with this way to particles to obtain a granulated material serving as bone substitute material.

3. Method according to claim 2, wherein the synthetic ceramic is produced in form of a plate, the surface of which on at least one plate side being provided with a regular or irregular grooved pattern, and wherein the plate is fractured along the grooves into particles, thereby obtaining the granulated material to serve as bone substitute material.

4. The method according to claim 2, wherein the synthetic ceramic is produced in form of a block which has a three-dimensional regular or irregular grid of perforation lines, along which the block is fractured into particles, thereby obtaining the granulated material to serve as bone substitute material.

5. The method according to claim 2, wherein the synthetic ceramic is produced in the form of a grid body with regularly or irregularly extending grid elements, and wherein the grid body is fractured into particles, thereby providing the granulated material to serve as bone substitute material.

6. The method according to claim 5, wherein said grid body is produced as a two-dimensional grid in sheet form.

7. The method according to claim 2, wherein the synthetic ceramic is produced in block or sheet form, and wherein said block or sheet formed synthetic ceramic is transformed into a granulated material, serving as the bone substitute material, by fracturing it on a first sieve with a first mesh size, thereby producing fractured particles, and then forwarding those fractured particles which have been passed through the first sieve to a second sieve with a second mesh size which is smaller than the first mesh size, wherein the particles selected from the second sieve are used as the granulated material.

8. The method of claim 2, wherein synthetic ceramic is fractured to particles having at least partial or, preferably, allover irregular outer surfaces.

9. The use/method according to one of claims 1 to 8, wherein the granular bone substitute material consists of particles having an average diameter of at least 0.5 mm and preferably a maximum of 6 mm.

10. Implant, in particular spinal implant for the insertion into an intervertebral space, having a hollow space, in which bone substitute material produced according to a method of one of claims 2-9 is received.

## Patentansprüche

1. Verwendung einer makroporösen Synthetik-Keramik, die hergestellt ist, indem Partikel aus einer organischen Komponente in einen Behälter gegeben werden, die Partikel einem Thermoformungsprozess unterzogen werden, wodurch die Partikel zu einer Monoblock-Struktur von miteinander verbundenen Partikeln mit dazwischen liegenden Hohlräumen zusammengeschmolzen werden, die Monoblock-Struktur mit einer Kalziumphosphat-Suspension benetzt wird, wodurch die Zwischenräume in der Monoblock-Struktur ausgefüllt werden, die Flüssigkeit aus der Suspension an der Monoblock-Struktur entfernt wird, die Monoblock-Struktur unter Ausbildung eines Keramik-Materials in Form eines Verbundes von miteinander verbundenen Hohlkörpern entfernt wird, deren Hohlräume durch Makroporen verbunden sind, und das Keramik-Material, um eine makroporöse Synthetik-Keramik zu erhalten, gesintert wird, zum Herstellen von granularem Knochenersatzmaterial.

2. Verfahren zum Herstellen von Knochenersatzmaterial, bei dem Partikel aus einer organischen Komponente in einen Behälter gegeben werden, die Partikel einem Thermoformungsprozess unterzogen werden, wodurch die Partikel zu einer Monoblock-Struktur von miteinander verbundenen Partikeln mit dazwischen liegenden Hohlräumen zusammengeschmolzen werden, die Monoblock-Struktur mit einer Kalziumphosphat-Suspension benetzt wird, wodurch die Zwischenräume in der Monoblock-Struktur gefüllt werden, die Flüssigkeit aus der Suspension an der Monoblock-Struktur entfernt wird, die Monoblock-Struktur unter Ausbildung eines Keramik-Materials in Form eines Verbundes von miteinander verbundenen Hohlkörpern entfernt wird, deren Hohlräume durch Makroporen verbunden sind, das Keramik-Material gesintert wird, so dass eine makroporöse Synthetik-Keramik gebildet wird, und die auf diese Art gewonnene Synthetik-Keramik zu Partikeln gebrochen wird, um ein als Knochenersatzmaterial dienendes Granulatmaterial zu erhalten.

3. Verfahren nach Anspruch 2, wobei die Synthetik-Keramik in Form einer Platte hergestellt wird, deren Oberfläche auf wenigstens einer Seite der Platte mit einem regelmäßigen oder unregelmäßigen Rillenmuster versehen ist, und wobei die Platte entlang der Rillen zu Partikeln zerbrochen wird, wodurch das als Knochenersatzmaterial dienende Granulatmaterial gewonnen wird.

4. Verfahren nach Anspruch 2, wobei die Synthetik-Keramik in Form eines Blocks hergestellt wird, der ein dreidimensionales regelmäßiges oder unregelmäßiges Gitter aus Perforationslinien aufweist, entlang derer der Block zu Partikeln zerbrochen wird, wodurch das als Knochenersatzmaterial dienende Granulatmaterial gewonnen wird.

5. Verfahren nach Anspruch 2, wobei die Synthetik-Keramik in Form eines Gitter-Körpers mit regelmäßig oder unregelmäßig verlaufenden Gitterelementen hergestellt wird, und wobei der Gitter-Körper zu Partikeln zerbrochen wird, wodurch das als Knochenersatzmaterial dienende Granulatmaterial ausgebildet wird.

6. Verfahren nach Anspruch 5, wobei der Gitter-Körper als ein zweidimensionales Gitter in Plattenform hergestellt wird.

7. Verfahren nach Anspruch 2, wobei die Synthetik-Keramik in Blockform oder Plattenform hergestellt wird, und wobei die block- oder plattenförmige Synthetik-Keramik in ein als Knochenersatzmaterial dienendes Granulatmaterial umgewandelt wird, indem sie auf einem ersten Sieb mit einer ersten Maschengröße gebrochen wird, wodurch gebrochene Partikel hergestellt werden, und indem diese gebrochenen Partikel, die durch das erste Sieb gefallen sind, zu einem zweiten Sieb mit einer zweiten Maschengröße, die kleiner ist als die erste Maschengröße, weitergeleitet werden, wobei die von dem zweiten Sieb erfassten Partikel als das Granulatmaterial verwendet werden.

8. Verfahren nach Anspruch 2, wobei eine Synthetik-Keramik in Partikel gebrochen wird, die mindestens teilweise oder bevorzugt über die ganze Fläche unregelmäßige Außenflächen aufweisen.

9. Verwendung bzw. Verfahren nach einem der Ansprüche 1 bis 8, wobei das granulare Knochenersatzmaterial aus Partikeln mit einem durchschnittlichen Durchmesser von mindestens 0,5 mm und bevorzugt von maximal 6 mm besteht.

10. Implantat, insbesondere Wirbelsäulenimplantat zum Einsetzen in einen Wirbelzwischenraum, mit einem Hohlraum, in welchem Knochenersatzmaterial aufgenommen ist, das nach einem Verfahren gemäß einem der Ansprüche 2 bis 9 hergestellt ist.

## Revendications

1. Utilisation d'une céramique synthétique macroporeuse qui est produite en introduisant des particules d'un composé organique dans un conteneur, en soumettant les particules à un processus de thermoformage, fusionnant ainsi les particules pour obtenir une structure monobloc de particules interconnectées avec des espaces creux entre elles, en imprégnant la structure monobloc d'une suspension de phosphate de calcium, comblant ainsi les espaces dans la structure monobloc, en enlevant le liquide de la suspension sur la structure monobloc, en enlevant la structure monobloc pour former un matériau céramique sous la forme d'un composé de corps creux interconnectés, dont les espaces creux sont connectés par des macropores, et en frittant le matériau céramique de façon à obtenir la céramique synthétique macroporeuse, pour produire un matériau substitut osseux en granules.

2. Procédé de fabrication d'un matériau substitut osseux, consistant à : introduire des particules d'un composé organique dans un conteneur, soumettre les particules à un processus de thermoformage, fusionnant ainsi les particules pour obtenir une structure monobloc de particules interconnectées avec des espaces creux entre elles, imprégner la structure monobloc d'une suspension de phosphate de calcium, comblant ainsi les espaces dans la structure monobloc, enlever le liquide de la suspension sur la structure monobloc, enlever la structure monobloc pour former un matériau céramique sous la forme d'un composé de corps creux interconnectés, dont les espaces creux sont connectés par des macropores, fritter le matériau céramique de façon à obtenir une céramique synthétique macroporeuse, et fracturer la céramique synthétique obtenue par ce procédé en particules pour obtenir un matériau granulé servant de matériau substitut osseux.

3. Procédé selon la revendication 2, dans lequel la céramique synthétique est produite sous la forme d'un plateau, dont la surface sur au moins une des faces du plateau est dotée d'un motif rainuré régulier ou irrégulier, et dans lequel le plateau est fracturé en particules suivant les rainures, fournissant ainsi le matériau granulé devant servir de matériau substitut osseux.

4. Procédé selon la revendication 2, dans lequel la céramique synthétique est produite sous la forme d'un bloc qui a un quadrillage tridimensionnel régulier ou irrégulier de lignes de perforation, suivant lesquelles le bloc est fracturé en particules, fournissant ainsi le matériau granulé devant servir de matériau substitut osseux.

5. Procédé selon la revendication 2, dans lequel la céramique synthétique est produite sous la forme d'un corps de quadrillage avec parties quadrillées prolongées de façon régulière ou irrégulière, et dans lequel le corps de quadrillage est fracturé en particules, fournissant ainsi le matériau granulé devant servir de matériau substitut osseux.

6. Procédé selon la revendication 5, dans lequel ledit corps de quadrillage est produit en tant que quadrillage bidimensionnel sous forme de feuille.

7. Procédé selon la revendication 2, dans lequel la céramique synthétique est produite sous forme de bloc ou de feuille, et dans lequel ladite céramique synthétique sous forme de bloc ou de feuille est transformée en un matériau granulé, servant de matériau substitut osseux, en la fracturant sur un premier crible avec une première largeur de mailles, produisant ainsi des particules fracturées, et en renvoyant ensuite ces particules fracturées qui ont été passées au premier crible vers un second crible avec une seconde largeur de mailles qui est inférieure à la première largeur de mailles, dans lequel les particules sélectionnées dans le second crible sont utilisées comme matériau granulé.

8. Procédé selon la revendication 2, dans lequel la céramique synthétique est fracturée en particules ayant une surface externe au moins partiellement ou, de préférence, totalement irrégulière.

9. Utilisation/procédé selon l'une des revendications 1 à 8, dans laquelle/lequel le matériau substitut osseux en granules est constitué par des particules ayant un diamètre moyen d'au moins 0,5 mm et de préférence d'un maximum de 6 mm.

10. Implant, en particulier implant rachidien pour insertion dans un espace intervertébral, ayant un espace creux, dans lequel le matériau substitut osseux produit selon un procédé de l'une des revendications 2-9 est recueilli.
